# EUROPEAN PATENT SPECIFICATION

(11) **EP 1 022 337 B1**
(45) Date of publication and mention of the grant of the patent: **26.07.2006**
(21) Application number: 00100129.6
(22) Date of filing: 07.01.2000
(51) Int. Cl.: C12N 15/24, C12N 15/70, C12N 15/72

(54) **Plasmids, their construction and their use in the manufacture of interleukin-4 and interleukin-4 muteins**
Plasmide, deren Konstruktion und deren Verwendung zur Herstellung von Interleukin-4 und Interleukin-4 Muteine
Plasmides,leur construction et leur utilisation pour la préparation d'interleukine-4 et de mutéines d'interleukine-4

(30) Priority: 20.01.1999 EP 99100967
(43) Date of publication of application: 26.07.2000
(73) Proprietor: Bayer HealthCare AG, 51368 Leverkusen (DE)
(72) Inventor: Apeler, Heiner, Dr., 42111 Wuppertal (DE); Wehlmann, Hermann, Dr., 42349 Wuppertal (DE)

(56) References cited:
- EP-A- 0 241 446
- WO-A-96/08572
- WO-A-98/03654
- US-A- 4 689 406
- US-A- 5 362 646
- PTITSYN L.R. AND AL'TMAN I.B.: "Recombinant Escherichia coli strains provide high-level expression of human Interleukin-3 and Interleukin-4" BULLETIN OF EXPERIMENTAL BIOLOGY AND MEDICINE, vol. 119, no. 1, January 1995 (1995-01), pages 77-79, XP002106664
- MAKRIDES S.C.: "Strategies for achieving high-level expression of genes in Escherichia coli" MICROBIOLOGICAL REVIEWS, vol. 60, no. 3, 1 September 1996 (1996-09-01), pages 512-538, XP002095235 ISSN: 0146-0749
- HANNIG G. ET AL.: "Strategies for optimizing heterologous protein expression in Escherichia coli" TRENDS IN BIOTECHNOLOGY, vol. 16, no. 2, 1 February 1998 (1998-02-01), pages 54-60, XP004107042
- OLINS P.O. ET AL.: "The T7 phage gene 10 leader RNA, a ribosome-binding site that dramatically enhances the expression of foreign genes in Escherichia coli" GENE, vol. 73, 1988, pages 227-235, XP002105448
- STUDIER F.W. ET AL.: "Use of T7 RNA polymerase to direct expression of cloned genes" METHODS IN ENZYMOLOGY, vol. 185, 1 January 1990 (1990-01-01), pages 60-89, XP000647676
- YOUNG YANG ET AL.: "Production, purification and immuno-modulatory actions of E.coli-derived recombinant human interleukin 4" KOREAN BIOCHEMICAL JOURNAL, vol. 25, no. 1, 1992, pages 66-72, XP002106663
- LEVINE A.D. ET AL.: "High level expression and refolding of mouse Interleukin 4 synthesized in Escherichia coli" THE JOURNAL OF BIOLOGICAL CHEMISTRY, vol. 270, no. 13, 31 March 1995 (1995-03-31), pages 7445-7452, XP002106665
- HANSSENS F. ET AL.: "Expression, renaturation and purification of murine Interleukin 4 from E. coli" MEDELINGEN VAN DE FACULTEIT LANDBOUWWETENSCHAPPEN UNIVERSITEIT GENT, vol. 57, no. 4b, 1992, pages 2053-2061, XP002107061

## Description

### Field of the Invention

The present invention relates to the construction and use of expression plasmids in the manufacture of recombinant interleukin-4 (IL-4) and interleukin-4 muteins.

### Background of the Invention

Mature human interleukin-4 (IL-4) is composed of 129 amino acids with 50% homology to mouse IL-4. IL-4 is the only cytokine known to direct the differentiation of T helper cells to a T_{H2} phenotype (Mosmann and Sad, Immunol. Today 17, 138 - 146, 1996). IL-4 signals on lymphocytes and other cells through a heterodimeric complex of two cytokine receptors, the IL-4Rα and the common γ-chain (γc). Antagonistic IL-4 mutants have been described (Kruse et al., EMBO J. 11, 3237 - 3244, 1992). Three amino acids close to the C-terminus (R121, Y124 and S125) are important for binding to the γc-chain. The introduction of Asp (D) into these positions blocks receptor dimerization and transmembrane signaling.

The interleukin-4 double mutein (IL-4 DM) is an IL-4 variant with 2 amino acid changes in position 121 and 124 termed IL-4 R121D Y124D. IL-4 DM is able to block both IL-4 and IL-13 activities. In contrast to all single site mutants no residual agonistic activity has ever been found for this mutein. It is believed that these antagonistic properties of IL-4 DM are useful for the treatment of diseases which involve T_{H2} development and/or IgE production (Ryan, J. Allergy Clin. Immunol. 99, 1 - 5, 1997).

As described in various publications, procaryotic organisms can be used to produce recombinant IL-4 and IL-4 muteins. Unfortunately, the described systems have a number of drawbacks (low expression level, low stability of the expression vector) which make large scale production of IL-4 and IL-4 muteins impossible or economically unfeasible. PTITSYN L.R. and AL'TMAN IB.: BULLETIN OF EXPERIMENTAL BIOLOGY AND MEDICINE, vol. 119, no. 1, January 1995, pages 77-79, is titled 'Recombinant *Escherichia coli* strains provide high-level expression of human Interleukin-3 and Interleukin-4,'The subject matter of the present invention differs from this citation that an E coli T5 promotor is concerned in the operon claimed. Unites States Patent 4,689,409 refers to enhancement of microbial expression of polypeptides and MAKRIDES S.C.: MICROBIOLOGICAL REVIEWS, vol. 60, no. 3,1 September 1996, pages 512-538, relates to 'Strategies for achieving high-level expression of genes in *Escherichia coli'.*

The main criteria for an efficient and safe expression system are:
- high product yield
- regulatable stable expression
- stability of the expression vector

Several features of an expression plasmid are important for the criteria listed above (Hannig et al., TIBTECH. 16, 54 - 60, 1998). These are:
- Promoter
- Ribosomal binding site (rbs)
- Codon usage of the corresponding gene
- Transcriptional terminator
- Resistance gene
- Regulation of expression
- Origin of replication (ori)

### Summary of the Invention

Expression plasmids for IL-4 and IL-4 muteins with modifications in all of the relevant elements for an efficient and safe expression system were generated. The quality and suitability of the corresponding expression system was ranked mainly according to the following criteria:
- Yield of IL-4 and IL-4 muteins
- Plasmid stability
- Maintenance of induction capability

The object of the present invention is, therefore, to make available a process for the construction and use of expression plasmids in the large scale manufacture of recombinant interleukin-4 (IL-4) and interleukin-4 muteins. In addition the newly developed host/vector system should be well suited for the expression of other proteins (cytokines, growth factors, soluble receptors, antibodies etc.).

Surprisingly, it has been found that bacteria transformed with plasmids according to the present invention give expression rates, plasmid and expression stability values many times higher than those observed after transforming the identical hosts with plasmids known in the art. Therefore, the plasmids of this invention are far more useful for the preparation of recombinant interleukin-4 and interleukin-4 muteins than all plasmids previously known.

The newly developed vector system contains the following elements:

### T5 promoter

The E. coli phage T5 promoter together with two lac operator sequences is derived from the pQE30 plasmid (Qiagen) belonging to the pDS family of plasmids (Bujard et al., Methods Enzymol. 155, 416 - 433, 1987 and Stüber et al., Immunological Methods, I. Lefkovits and B. Pernis, eds., Academic Press, Inc., Vol. IV, 121 - 152, 1990).

### T7 g10 ribosomal binding site

The ribosomal binding site (rbs) is derived from the region upstream from gene 10 of the phage T7 (T7 g 10 leader). Gene 10 of phage T7 codes for the coat protein, which is the major protein expressed after T7 infection. The T7 g10 rbs was obtained from the vector pET-9a (Studier et al., Methods Enzymol. 185, 60 - 89, 1990). The T7 g10 leader spans a region of about 100 bp (Olins et al., Gene 227 - 235, 1988). In the final expression construct the region upstream of the XbaI site is deleted. The T7 g10 leader sequence now spans 42 bp and harbours one base exchange from G to A in position 3638 of the preferred plasmid.

### Codon usage of the natural IL-4 sequence

As an effective measure of synonymous codon usage bias, the codon adaptation index (CAI) can be useful for predicting the level of expression of a given gene (Sharp et al., Nucleic Acids Res. 15, 1281 - 1295, 1987 and Apeler et al., Eur. J. Biochem. 247, 890 - 895, 1997). The CAI is calculated as the geometric mean of relative synonymous codon usage (RSCU) values corresponding to each of the codons used in a gene, divided by the maximum possible CAI for a gene of the same amino acid composition. RSCU values for each codon are calculated from very highly expressed genes of a particular organism, e.g. E. coli, and represent the observed frequency of a codon divided by the frequency expected under the assumption of equal usage of the synonymous codons for an amino acid. Highly expressed genes, e.g. genes encoding ribosomal proteins, have generally high CAI values ≥ 0.46. Poorly expressed genes like lacl and trpR in E. coli have low CAI values ≤ 0.3.

The calculated E. coli CAI value for the natural IL-4 sequence is 0.733. This means that the natural gene should be well-suited for high level expression in E. coli. Nevetheless a synthetic gene with optimal E. coli codon usage (CAI value = 1) has the potential to further increase the expression level. Therefore synthetic IL-4 and IL-4 mutein genes were designed and cloned.

### Transcriptional terminator

A T7 DNA fragment containing the transcription terminator Tφ is derived from the vector pET-9a (Studier et al., Methods Enzymol. 185, 60 - 89, 1990). Transcriptional terminators determine the points where the mRNA-RNA polymerase- DNA complex dissociates, thereby ending transcription. The presence of a transcriptional terminator at the end of a highly expressed gene has several advantages: they minimize sequestering of RNA polymerase that might be engaged in unnecessary transcription, they restrict the mRNA length to the minimal, thus limiting energy expense,
as strong transcription may interfere with the origin of replication, a transcriptional terminator increases plasmid stability due to copy number maintenance (Balbas and Bolivar, Methods Enzymol. 185, 14 -37, 1990).

### Resistance gene

The kan resistance gene is derived from the vector pET-9a (Studier et al., Methods Enzymol. 185, 60 -89, 1990). Originally, this is the kan gene of Tn903 from the vector pUC4KISS (Barany, Gene 37, 111 - 123, 1985). In the preferred plasmid the kan gene and the IL-4 and IL-4 mutein gene have opposite orientations, so there should not be an increase in kan gene product after induction due to read-through transcription from the T5 promoter. Kanamycin was chosen as selective marker because it is the preferred antibiotic for GMP-purposes. In addition, kan gene based vectors are more stable than ampicillin resistant (bla) plasmids. Ampicillin selection tends to be lost in cultures as the drug is degraded by the secreted β-lactamase enzyme. The mode of bacterial resistance to kanamycin relies upon an aminoglycoside phosphotransferase that inactivates the antibiotic.

### Regulation of expression

Controlled gene expression is absolutely necessary for the set-up of a stable plasmid system, particularly if the protein of interest is deleterious to the host cell. The preferred plasmid uses a lac-based inducible system consisting of a lac repressor gene (lacI) and two synthetic lac operator sequences fused downstream to the E. coli phage T5 promoter. The lacI^{q} promoter and the lad structural gene were isolated from the vector pTrc99A (Amann et al., Gene 69, 301 - 315, 1988). I^{q} is a promoter mutation which leads to overproduction of the lad repressor. The wild-type lac repressor is a tetrameric molecule comprising four identical subunits of 360 amino acids each. The lac repressor tetramer is a dimer of two functional dimers. The four subunits are held together by a four-helix bundle formed from residues 340 - 360. Due to the isolation of the lacI gene from the vector pTrc99A by a NarI cut the residues beyond amino acid 331 are deleted and 10 amino acids not normally encoded in the lacI gene are added. It is known that mutations or deletions that occur in the C-terminal part of lad, beyond amino acid 329, result in functional dimers that appear phenotypically similar to the wild-type repressor (Pace et al., TIBS 22, 334 - 339, 1997).

### Origin of replication (ori)

The origin of replication (ori) of the preferred plasmid is derived from the vector pET-9a, the ori of which originates from pBR322. The preferred plasmid therefore carries the pMB1 (ColEl) replicon. Plasmids with this replicon are multicopy plasmids that replicate in a 'relaxed' fashion. A minimum of 15 - 20 copies of plasmid are maintained in each bacterial cell under normal growth conditions. The actual number for the preferred plasmid is within this range. Replication of the Co1E1-type ori is initiated by a 555-nucleotide RNA transcript, RNA II, which forms a persistent hybrid with its template DNA near the ori. The RNA II-DNA hybrid is then cleaved by RNase H at the ori to yield a free 3'OH that serves as a primer for DNA polymerase I. This priming of DNA synthesis is negatively regulated by RNA I, a 108-nucleotide RNA molecule complementary to the 5'end of RNA II.I nteraction of the antisense RNA I with RNA II causes a conformational change in RNA II that inhibits binding of RNA II to the template DNA and consequently prevents the initiation of plasmid DNA synthesis. The binding between RNAs I and II is enhanced by a small protein of 63 amino acids (the Rop protein, Repressor of primer), which is encoded by a gene located 400 nucleotides downstream from the origin of replication (Sambrook et al., Molecular Cloning, Cold Spring Harbor, 1989). Deletion of the rop gene leads to an increase in copy number and due to a gene dosage effect to enhanced expression levels of the plasmid encoded heterologous gene. This observation was also made for the IL-4 expression vectors tested. But it turned out that rop⁻-plasmids are instable and lost very rapidly during fermentation under non-selective conditions. Therefore the replicon of the preferred plasmid contains the rop gene to ensure high plasmid stability. The preferred plasmid lacks the mob gene that is required for mobilization and is therefore incapable of directing its own conjugal transfer from one bacterium to another.

In the preferred plasmid all elements not necessary for plasmid replication, resistance and regulatable expression were deleted.

For example, a natural interleukin-4 or interleukin-4 mutein gene can be used instead of a synthetic one with optimized E. coli codon usage. The preferred transcription terminator is Tφ, but other terminators like rmB T2 or aspA can also be used.

The methods employed in the course of the establishment of the expression system are given below.

### MATERIALS AND METHODS

### Enzymes

Restriction endoculeases, calf intestinal alkaline phosphatase, T4 polynucleotide kinase and T4 DNA ligase were purchased from Boehringer Mannheim and GIBCO-BRL and used as recommended by the supplier.

### Recombinant DNA methods

Standard cloning procedures have been described in Sambrook et al. (Molecular Cloning, Cold Spring Harbor, 1989). Transformations were performed according to M. Scott (Seed and Aruffo, Proc. Natl. Acad. Sci. USA 84, 3365 - 3369, 1987). As hosts for transformations the E. coli strains DH5α (GIBCO BRL) and W3110 (ATCC 27325) were primarily used. The genotype of W3110 is K12, F⁻, [N(rrnD-rrnE)]λ⁻.

Large scale isolations of plasmid DNA were carried out with Qiagen-tips (Qiagen). Extraction of DNA fragments from agarose gels was performed using Jetsorb (Genomed) as recommended by the supplier.

Oligonucleotides for site directed mutagenesis, PCR reactions and sequencing were obtained from MWG Biotech, Pharmacia Biotech or GIBCO BRL.

The mutagenesis experiments were carried out by the method of Deng and Nickoloff (Deng and Nickoloff, Anal. Biochem. 200, 81 - 88, 1992) using the 'Unique Site Elimination Mutagenesis' system from Pharmacia Biotech. The primer used for the recreation of the T7 g10 rbs has the following sequence:
5'TCAATTGTGAGCGGATAACAATTTCACACATCTAGAAATAAGTTT AACTTTAAGAA3' (Seq.1).

All constructs and DNA sequences were confirmed using Dye Terminator Cycle Sequencing with AmpliTaq DNA Polymerase, FS on an ABI 373A sequencer (Applied Biosystems).

The invention is explained in more detail by the following examples, figures and sequences information:

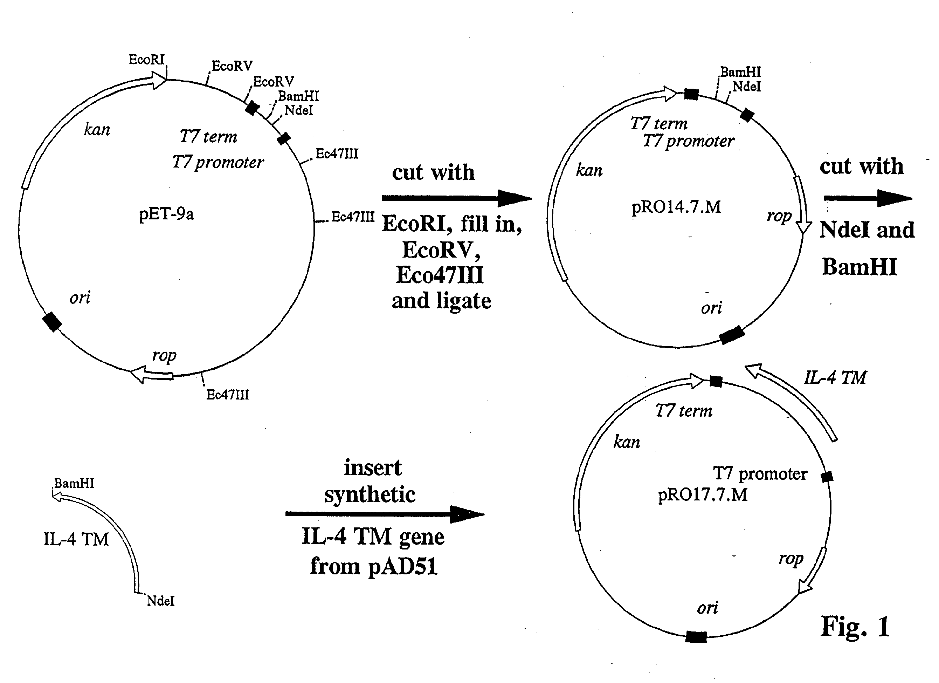

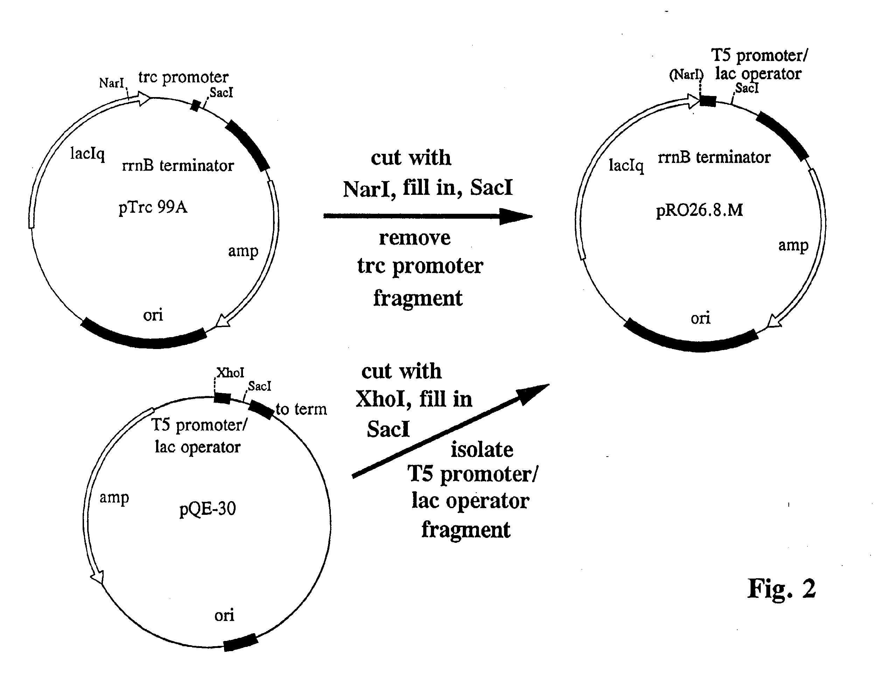

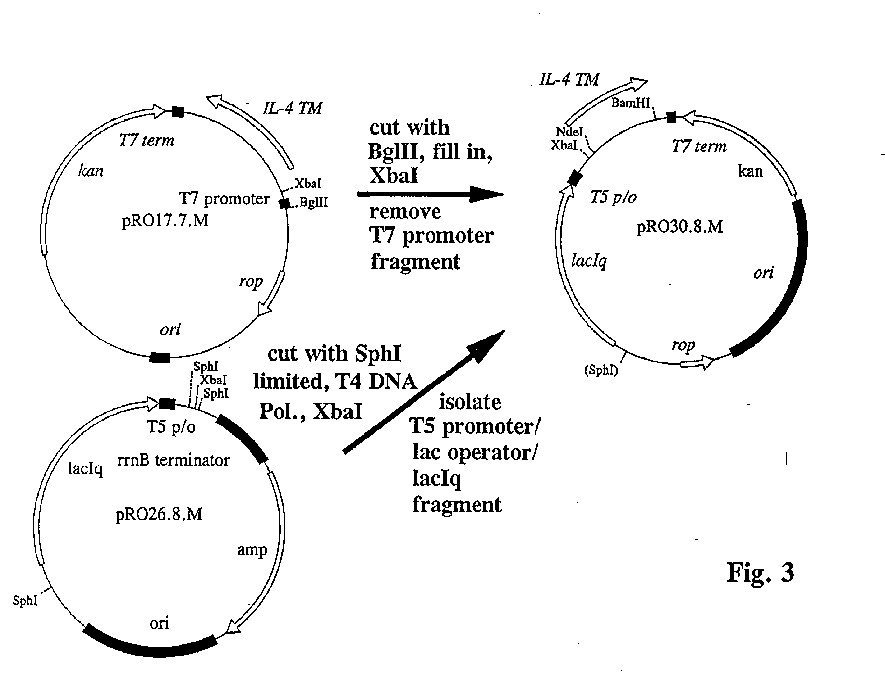

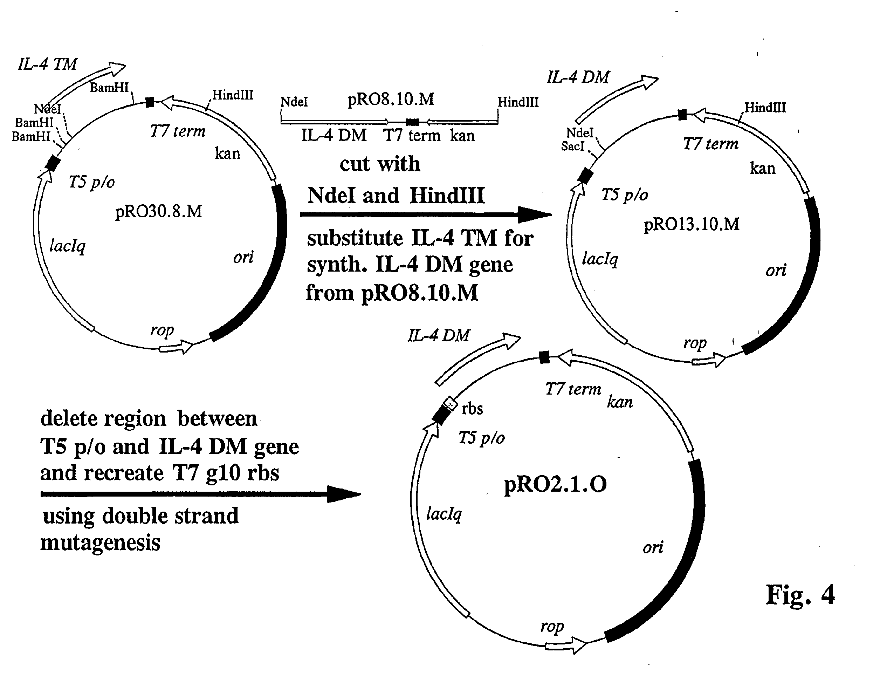

- FIG.1 to FIG. 4:: Construction of the preferred expression plasmid. (Abbreviations: IL-4 TM, IL-4 triple mutein; IL-4 DM, IL-4 double mutein).

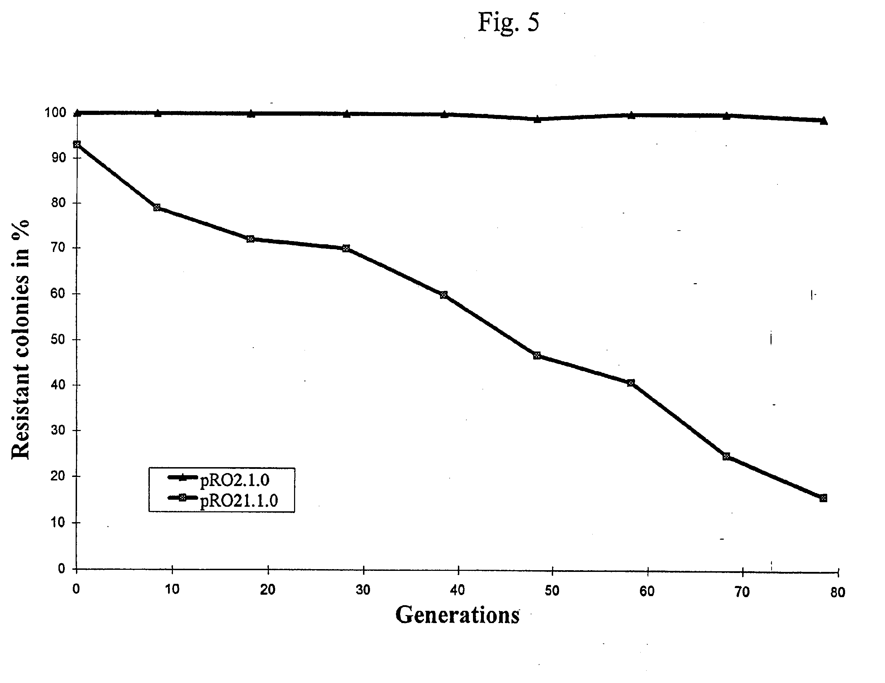

- FIG. 5:: Plasmid stability of the IL-4 mutein expression vectors pRO21.1.O and pRO2.1.O. The vector pRO2.1.O remains fully stable over 78 generations without antibiotic selection. In contrast the expression vector pRO21.1.O, which is based on the commercially available plasmid pET-30a (Novagen), is lost very rapidly. Only 16% of the colonies contain the plasmid after 78 generations without kanamycin selection.

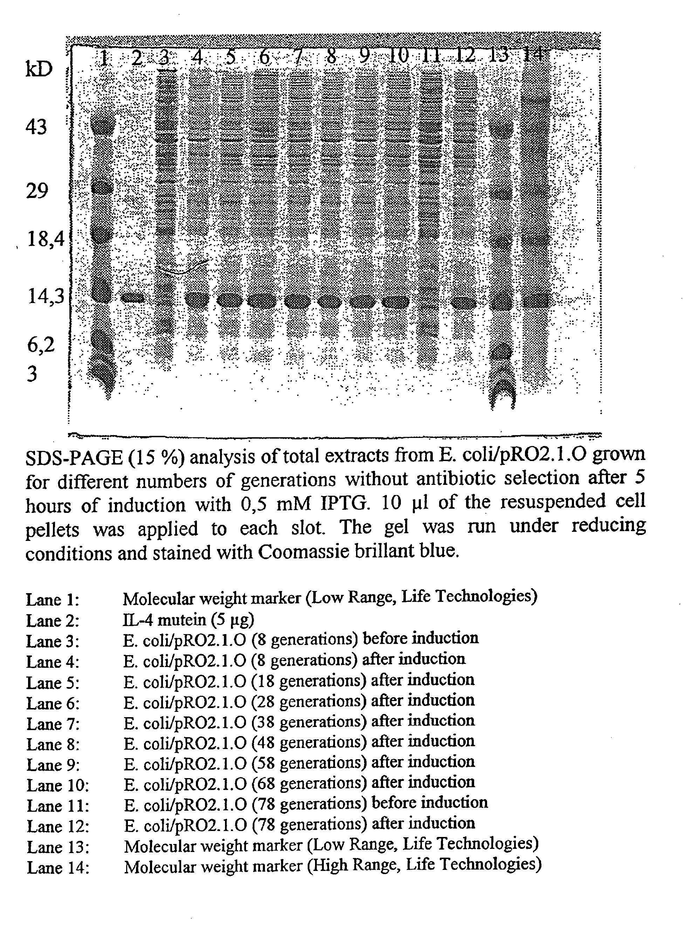

- FIG. 6:: Maintenance of induction and expression capability of the preferred IL-4 and IL-4 mutein expression vector.

### EXAMPLES

### Example 1

### Plasmid stability test

The plasmid stability tests were always started with a culture frozen in liquid nitrogen. The OD₆₀₀ of the thawed culture was determined, the culture diluted up to 10⁻⁴ in PBS buffer and plated on LB agar plates without antibiotic.

1 ml of the thawed culture was inoculated into 100 ml of peptone medium (30 g Soya peptone, 20 g Yeast extract, 5 g KH₂PO₄, 20 g Glycerol, 1 g MgSO₄ x 7H₂O per liter, pH 7,0) and incubated at 37°C with 280 rpm for 24 hours.

The OD₆₀₀ of the grown culture was determined, the culture diluted up to 10⁻⁶ in PBS buffer and plated on LB agar plates without antibiotic to get well separated colonies.

100 µl from the grown culture were inoculated into 100 ml peptone medium and incubated at 37°C with 280 rpm for 24 hours. This procedure was repeated eight times.

100 well separated colonies from the LB plates were gridded onto LB plates with kanamycin (25 µg/ml) and LB plates without kanamycin and incubated at 37°C overnight. The percentage of resistant colonies was determined on every day.

The number of generations per day were calculated according to the following formula: log [OD₆₀₀ END /OD₆₀₀ BEG] /log 2.

For the expression studies 1 ml of a grown culture was diluted 100 fold into LB medium and handled as described (see Example 2).

### Example 2

### Expression

For small scale expression of interleukin-4 and interleukin-4 muteins cells were grown in LB medium (10 g Bacto tryptone, 5 g Yeast extract, 10 g NaCl per liter, pH 7,5) until OD600 reached 0.8 - 1.0. Expression was induced by addition of IPTG to a final concentration of 0,5 mM and incubation continued for 5 hours. Cells were harvested by centrifugation.

For SDS-PAGE analysis cell pellets were resuspended in SDS-PAGE loading buffer to a concentration of 1 OD600 unit/100 µl.

## Claims

1. A vector for the manufacture of IL-4 and IL-4 muteins in a strain of Escherichia coli, comprising in 5' to 3' order the following operatively linked elements: a regulatable promoter consisting of the E. coli phage T5 promoter and two lac operator sequences, a ribosome binding site from the E. coli phage T7 g10, a translational start codon, a structural gene for IL-4 or an IL-4 mutein and downstream of that structural gene one transcription terminator.

2. DNA construct which comprises the DNA sequence shown in Seq. ID. No. 2.

3. Escherichia coli transformed with the vector of claim 1 or with the DNA construct of claim 2.

4. Use of the vector according to claim 1 or of the DNA construct according to claim 2 in a method of preparation of IL-4 and IL-4 muteins.

## Patentansprüche

1. Vektor zur Herstellung von IL-4 und IL-4-Muteinen in einem Stamm von Escherichia coli, umfassend in 5'- zu 3'-Richtung die folgenden operativ gebundenen Elemente: einen regulierbaren Promotor, der aus dem E.-coli-Phagen-T5-Promotor und zwei lac-Operatorsequenzen besteht, eine Ribosomen-Bindungsstelle aus dem E.-coli-Phagen T7 g10, ein Translationsstartcodon, ein strukturelles Gen für IL-4 oder ein IL-4-Mutein und stromab von diesem strukturellen Gen einen Transkriptionsterminator.

2. DNA-Konstrukt, das die in Seq.-ID Nr. 2 gezeigte DNA-Sequenz umfasst.

3. Escherichia coli, transformiert mit dem Vektor nach Anspruch 1 oder mit dem DNA-Konstrukt nach Anspruch 2.

4. Verwendung des Vektors nach Anspruch 1 oder des DNA-Konstrukts nach Anspruch 2 in einem Verfahren zur Herstellung von IL-4 und IL-4-Muteinen.

## Revendications

1. Vecteur pour la production de IL-4 et de mutéines de IL-4 dans une souche d'Escherichia coli, comprenant, dans l'ordre de 5' à 3', les éléments liés de manière fonctionnelle suivants : un promoteur régulable consistant en le promoteur de phage T5 de E. coli et deux séquences d'opérateurs lac, un site de liaison ribosomale provenant de g10 du phage T7 de E. coli, un codon d'initiation de traduction, un gène structural pour la IL-4 ou une mutéine de IL-4 et, en aval de ce gène structural, un terminateur de transcription.

2. Produit d'assemblage d'ADN qui comprend la séquence d'ADN représentée dans la SEQ ID N° 2.

3. Escherichia coli transformée avec le vecteur de la revendication 1 ou avec le produit d'assemblage d'ADN de la revendication 2.

4. Utilisation du vecteur suivant la revendication 1 ou du produit d'assemblage d'ADN suivant la revendication 2, dans un procédé pour la préparation de IL-4 et de mutéines de IL-4.


### SEQUENCE LISTING

<110> Bayer AG
<120> PLASMIDS, THEIR CONSTRUCTION AND THEIR USE IN THE MANUFACTURE OF INTERLEUKIN-4 AND INTERLEUKIN-4 MUTEINS
<130> Plasmids for IL-4 muteins
<140>
   <141>
<160> 2
<170> PatentIn Ver. 2.0
<210> 1
   <211> 60
   <212> DNA
   <213> Unknown
<220>
   <223> Description of Unknown Organism: primer
<400> 1
   tcaattgtga gcggataaca atttcacaca tctagaaata attttgttta actttaagaa 60
<210> 2
   <211> 4202
   <212> DNA
   <213> Unknown
<220>
   <223> Description of Unknown Organism: Human
<400> 2
   ttagaaaaac tcatcgagca tcaaatgaaa ctgcaattta ttcatatcag gattatcaat 60
   accatatttt tgaaaaagcc gtttctgtaa tgaaggagaa aactcaccga ggcagttcca 120
   taggatggca agatcctggt atcggtctgc gattccgact cgtccaacat caatacaacc 180
   tattaatttc ccctcgtcaa aaataaggtt atcaagtgag aaatcaccat gagtgacgac 240
   tgaatccggt gagaatggca aaagcttatg catttctttc cagacttgtt caacaggcca 300
   gccattacgc tcgtcatcaa aatcactcgc atcaaccaaa ccgttattca ttcgtgattg 360
   cgcctgagcg agacgaaata cgcgatcgct gttaaaagga caattacaaa caggaatcga 420
   atgcaaccgg cgcaggaaca ctgccagcgc atcaacaata ttttcacctg aatcaggata 480
   ttcttctaat acctggaatg ctgttttccc ggggatcgca gtggtgagta accatgcatc 540
   atcaggagta cggataaaat gcttgatggt cggaagaggc ataaattccg tcagccagtt 600
   tagtctgacc atctcatctg taacatcatt ggcaacgcta cctttgccat gtttcagaaa 660
   caactctggc gcatcgggct tcccatacaa tcgatagatt gtcgcacctg attgcccgac 720
   attatcgcga gcccatttat acccatataa atcagcatcc atgttggaat ttaatcgcgg 780
   cctcgagcaa gacgtttccc gttgaatatg gctcataaca ccccttgtat tactgtttat 840
   gtaagcagac agttttattg ttcatgacca aaatccctta acgtgagttt tcgttccact 900
   gagcgtcaga ccccgtagaa aagatcaaag gatcttcttg agatcctttt tttctgcgcg 960
   taatctgctg cttgcaaaca aaaaaaccac cgctaccagc ggtggtttgt ttgccggatc 1020
   aagagctacc aactcttttt ccgaaggtaa ctggcttcag cagagcgcag ataccaaata 1080
   ctgtccttct agtgtagccg tagttaggcc accacttcaa gaactctgta gcaccgccta 1140
   catacctcgc tctgctaatc ctgttaccag tggctgctgc cagtggcgat aagtcgtgtc 1200
   ttaccgggtt ggactcaaga cgatagttac cggataaggc gcagcggtcg ggctgaacgg 1260
   ggggttcgtg cacacagccc agcttggagc gaacgaccta caccgaactg agatacctac 1320
   agcgtgagct atgagaaagc gccacgcttc ccgaagggag aaaggcggac aggtatccgg 1380
   taagcggcag ggtcggaaca ggagagcgca cgagggagct tccaggggga aacgcctggt 1440
   atctttatag tcctgtcggg tttcgccacc tctgacttga gcgtcgattt ttgtgatgct 1500
   cgtcaggggg gcggagccta tggaaaaacg ccagcaacgc ggccttttta cggttcctgg 1560
   ccttttgctg gccttttgct cacatgttct ttcctgcgtt atcccctgat tctgtggata 1620
   accgtattac cgcctttgag tgagctgata ccgctcgccg cagccgaacg accgagcgca 1680
   gcgagtcagt gagcgaggaa gcggaagagc gcctgatgcg gtattttctc cttacgcatc 1740
   tgtgcggtat ttcacaccgc aatggtgcac tctcagtaca atctgctctg atgccgcata 1800
   gttaagccag tatacactcc gctatcgcta cgtgactggg tcatggctgc gccccgacac 1860
   ccgccaacac ccgctgacgc gccctgacgg gcttgtctgc tcccggcatc cgcttacaga 1920
   caagctgtga ccgtctccgg gagctgcatg tgtcagaggt tttcaccgtc atcaccgaaa 1980
   cgcgcgaggc agctgcggta aagctcatca gcgtggtcgt gaagcgattc acagatgtct 2040
   gcctgttcat ccgcgtccag ctcgttgagt ttctccagaa gcgttaatgt ctggcttctg 2100
   ataaagcggg ccatgttaag ggcggttttt tcctgtttgg tcactgatgc ctccgtgtaa 2160
   gggggatttc tgttcatggg ggtaatgata ccgatgaaac gagagaggat gctcacgata 2220
   cgggttactg atgatgaaca tgcccggtta ctggaacgtt gtgagggtaa acaactggcg 2280
   gtatggatgc ggcgggacca gagaaaaatc actcagggtc aatgccagcg ctcatgagcc 2340
   cgaagtggcg agcccgatct tccccatcgg tgatgtcggc gatataggcg ccagcaaccg 2400
   cacctgtggc gccggtgatg ccggccacga tgcgtccggc gtagaggatc gagatccatt 2460
   tacgttgaca ccatcgaatg gtgcaaaacc tttcgcggta tggcatgata gcgcccggaa 2520
   gagagtcaat tcagggtggt gaatgtgaaa ccagtaacgt tatacgatgt cgcagagtat 2580
   gccggtgtct cttatcagac cgtttcccgc gtggtgaacc aggccagcca cgtttctgcg 2640
   aaaacgcggg aaaaagtgga agcggcgatg gcggagctga attacattcc caaccgcgtg 2700
   gcacaacaac tggcgggcaa acagtcgttg ctgattggcg ttgccacctc cagtctggcc 2760
   ctgcacgcgc cgtcgcaaat tgtcgcggcg attaaatctc gcgccgatca actgggtgcc 2820
   agcgtggtgg tgtcgatggt agaacgaagc ggcgtcgaag cctgtaaagc ggcggtgcac 2880
   aatcttctcg cgcaacgcgt cagtgggctg atcattaact atccgctgga tgaccaggat 2940
   gccattgctg tggaagctgc ctgcactaat gttccggcgt tatttcttga tgtctctgac 3000
   cagacaccca tcaacagtat tattttctcc catgaagacg gtacgcgact gggcgtggag 3060
   catctggtcg cattgggtca ccagcaaatc gcgctgttag cgggcccatt aagttctgtc 3120
   tcggcgcgtc tgcgtctggc tggctggcat aaatatctca ctcgcaatca aattcagccg 3180
   atagcggaac gggaaggcga ctggagtgcc atgtccggtt ttcaacaaac catgcaaatg 3240
   ctgaatgagg gcatcgttcc cactgcgatg ctggttgcca acgatcagat ggcgctgggc 3300
   gcaatgcgcg ccattaccga gtccgggctg cgcgttggtg cggatatctc ggtagtggga 3360
   tacgacgata ccgaagacag ctcatgttat atcccgccgt taaccaccat caaacaggat 3420
   tttcgcctgc tggggcaaac cagcgtggac cgcttgctgc aactctctca gggccaggcg 3480
   gtgaagggca atcagctgtt gcccgtctca ctggtgaaaa gaaaaaccac cctggctcga 3540
   gaaatcataa aaaatttatt tgctttgtga gcggataaca attataatag attcaattgt 3600
   gagcggataa caatttcaca catctagaaa taattttatt taactttaag aaggagatat 3660
   acatatgcac aaatgcgata tcaccctgca ggaaatcatc aaaaccctga attctctgac 3720
   cgaacagaaa accctgtgca ccgaactgac cgttaccgac atcttcgctg cttcgaaaaa 3780
   caccaccgaa aaagaaacct tctgccgtgc tgctaccgtt ctgcgtcagt tctactctca 3840
   ccacgaaaaa gacacccgtt gcctgggtgc taccgctcag cagttccacc gtcacaaaca 3900
   gctgatccgt ttcctgaaac gtctggaccg taacctgtgg ggtctggctg gtctgaacag 3960
   ctgcccggtt aaagaagcta accagtctac cctggaaaac ttcctggaac gtctgaaaac 4020
   catcatggac gaaaaagact ctaaatgctc ttcttaataa ggatccggct gctaacaaag 4080
   cccgaaagga agctgagttg gctgctgcca ccgctgagca ataactagca taaccccttg 4140
   gggcctctaa acgggtcttg aggggttttt tgctgaaagg aggaactata tccggataat 4200
   tc                                                                4202